# DEMANDE DE BREVET EUROPEEN

(11) **EP 2 608 095 A1**
(43) Date de publication de la demande: **26.06.2013**
(21) Numéro de dépôt: 12290397.4
(22) Date de dépôt: 16.11.2012
(51) Int. Cl.: G06F 19/00, G06F 9/50, F02D 28/00

(54) **Système et procédé de prédiction des émissions de polluants d'un vehicule avec calculs simultanes de la cinétique chimique et des emissions**

(30) Priorité: 20.12.2011 FR 1103991
(71) Demandeur: IFP Energies nouvelles, 92852 Rueil-Malmaison Cedex (FR)
(72) Inventeur: Bohbot, Julien, 91370 Verrieres le Buisson (FR); Velghe, Anthony, 92500 Rueil-Malmaison (FR)

(57) **Abrégé**

L'invention concerne un système de prédiction des émissions de polluants d'un véhicule équipé d'un moteur, comprenant plusieurs processeurs (3) alloués pour la détermination des émissions de polluants comprenant :
• un premier groupe de processeurs (1) dédié à l'exécution de calculs de la cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
• un deuxième groupe de processeurs (2) dédié à l'exécution de calculs des émissions de polluants, lesdits calculs permettant de déterminer les émissions de polluants à partir des quantités des composés chimiques calculées,
les exécutions des calculs par lesdits deux groupes de processeurs (1 ; 2) étant simultanées.

Le premier groupe de processeurs (1) est constitué d'un processeur superviseur global (4) et d'au moins une grappe de processeurs (5), ledit superviseur (4) permettant le stockage de données nécessaires pour les calculs et de répartir lesdits calculs sur lesdites grappes (5) de processeurs disponibles.

## Description

La présente invention concerne le domaine des simulations des émissions de polluants de véhicules, notamment automobiles.

Les contraintes économiques et environnementales sur la consommation de carburant et les émissions de polluants sont de plus en plus contraignantes pour les constructeurs automobiles. Par exemple, la norme EURO fixant la limite maximale des rejets de polluants des véhicules (CO₂, NOx, suies, particules et hydrocarbures imbrûlés) devient de plus en plus contraignante. Les constructeurs cherchent donc à optimiser leurs véhicules, dans le but de minimiser les émissions de polluants. Les simulations des émissions de polluants sont par conséquent de plus en plus utilisées afin de prévoir le comportement du moteur, sans avoir à réaliser de nombreuses et longues mesures expérimentales.

Afin de prédire l'émission des polluants des véhicules, la phase de post-oxydation (c'est-à-dire l'oxydation des gaz par une flamme de diffusion) dans la chambre de combustion est simulée grâce à une méthode de tabulation de la cinétique chimique de type FPI (Flame Prolongation of Intrinsic Low Dimensional Manifold pouvant être traduit par prolongation de la flamme dans le collecteur de faible dimension intrinsèque). Les tables utilisées pour ce type de modèle sont générées à partir d'un code informatique résolvant la cinétique chimique, par exemple le logiciel Chemkin ®, développé par Reaction Design ®. Cette tabulation détaille les quantités molaires de tous les composés chimiques présents dans une réaction, en fonction d'un schéma réactionnel donné et de conditions thermodynamiques. Puis, ces tables sont utilisées lors du calcul des émissions de polluants par un programme d'ordinateur, par exemple de type CFD (pour Computational Fluid Dynamics, pouvant être traduit par mécanique des fluides numérique), notamment avec le logiciel IFP-C3D ®, développé par IFP Energies Nouvelles.

Cependant, cette solution présente plusieurs inconvénients. D'abord, les méthodes de tabulation doivent prendre en compte le chemin thermodynamique et chimique, ce qui est très complexe pour la modélisation du moteur. Afin de contourner ce problème, des variables supplémentaires doivent être introduites dans les tables. Ce nombre de paramètres croissant entraine une augmentation des tailles des tables chimiques ce qui implique de produire des tables prenant un espace physique mémoire très important. De plus, lorsque les tables sont générées, certaines hypothèses physiques, notamment sur le type de réacteur chimique (c'est-à-dire l'enceinte dans laquelle la réaction chimique a lieu) utilisé sont faites. Les tables générées ayant comme hypothèse que les réacteurs chimiques sont à volume constant sont inutilisables pour les cas de réacteurs à volumes variables. Ce premier choix est fondamental pour la simulation de chambre de combustion à géométrie mobile. Cependant, lors des cycles moteurs, la réalité peut se rapprocher d'une hypothèse puis de l'autre. Par conséquent, les résultats des simulations peuvent être imprécis voire erronés.

Pour s'affranchir de ces difficultés, le développement d'un couplage direct entre les deux codes de calcul, par exemple des logiciels IFP-C3D ® et Chemkin ®, a été envisagé pour la simulation de la phase de post-oxydation des gaz brulés. Selon cette méthode, lors du couplage direct, la composition du mélange et les termes concernant les sources chimiques sont calculés pendant le calcul des émissions de polluants. Lorsqu'on exécute le code de calcul des émissions de polluants, la composition du mélange est évaluée par un calcul direct de la cinétique chimique à partir des conditions thermodynamiques locales données. La partie de calcul des émissions de polluants reçoit les résultats chimiques et les incorpore dans les équations. Ainsi, la simulation peut se poursuivre. De cette façon, la connaissance du chemin thermodynamique n'est plus nécessaire, ce qui simplifie la modélisation chimique.

Toutefois, le couplage direct nécessite un calcul de cinétique chimique à chaque itération en temps et pour toutes les mailles qui brûlent, ceci va entrainer un surcoût de calcul qu'il faut réduire au maximum pour rendre compatible son utilisation avec les contraintes industrielles.

La présente invention permet de prédire les émissions de polluants d'un véhicule équipé d'un moteur à combustion, en réalisant les calculs de cinétique chimique simultanément aux calculs des émissions de polluants, les calculs de cinétique étant distribués puis exécutés simultanément par plusieurs processeurs, cela est permis par une architecture en parallèle des processeurs de calcul. Le système selon l'invention permet de gérer automatiquement la distribution des calculs de cinétique chimique.

### Description de l'invention

L'invention concerne un système de prédiction des émissions de polluants d'un véhicule équipé d'un moteur, comprenant plusieurs processeurs alloués pour la détermination des émissions de polluants comprenant :
- un premier groupe de processeurs dédié à l'exécution de calculs de la cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
- un deuxième groupe de processeurs dédié à l'exécution de calculs des émissions de polluants, lesdits calculs permettant de déterminer les émissions de polluants à partir des quantités des composés chimiques calculées,
les exécutions des calculs par lesdits deux groupes de processeurs étant simultanées.

Ledit premier groupe de processeurs est constitué d'un processeur superviseur global et d'au moins une grappe de processeurs, ledit superviseur permettant le stockage de données nécessaires pour les calculs et de répartir lesdits calculs sur lesdites grappes de processeurs disponibles.

Selon l'invention, ladite au moins une grappe de processeurs est constituée d'un processeur superviseur local et d'au moins un processeur esclave, ledit superviseur local permettant de stocker les données et de répartir lesdits calculs entre lesdits processeurs esclaves, et chaque processeur esclave étant apte à réaliser des calculs de cinétique chimique pour une portion du modèle dudit moteur.

En outre, ledit système comprend des premiers moyens de communication entre les deux groupes de processeurs, lesdits moyens de communication permettant de transférer les données issues du calcul des émissions de polluants vers le premier groupe de processeurs ainsi que les résultats des calculs de cinétique chimique vers ledit deuxième groupe de processeurs.

De plus, ledit système comprend des deuxièmes moyens de communication entre ledit superviseur global et lesdites grappes de processeurs, lesdits moyens de communication permettant de transférer les données nécessaires pour les calculs depuis ledit superviseur global vers lesdites grappes ainsi que les résultats desdites simulations depuis lesdites grappes vers ledit superviseur global.

Avantageusement, ledit système comprend des troisièmes moyens de communication entre lesdits superviseurs locaux et lesdits processeurs esclaves, lesdits moyens de communication permettant de transférer les données nécessaires pour la simulation depuis ledit superviseur local vers lesdits processeurs esclaves ainsi que les résultats desdits calculs desdits processeurs esclaves vers ledit superviseur local.

De préférence, lesdits calculs de la cinétique chimique sont exécutés par un logiciel de cinétique chimique, apte à être exécuté par le premier groupe de processeurs et lesdits calculs des émissions de polluants sont exécutés par un logiciel de calculs, apte à être exécuté par le deuxième groupe de processeurs.

Selon un mode de réalisation préférentiel de l'invention, le couplage des calculs des deux groupes de processeurs utilise une interface de passage de messages MPI.

De manière avantageuse lesdits moyens de communications sont réalisés par des fonctions choisies dans la librairie de fonctions de l'interface MPI.

De préférence, chaque groupe de processeurs constitue un communicateur global.

Avantageusement, chaque grappe de processeurs constitue un communicateur de chimie.

L'invention concerne en outre un procédé de prédiction des émissions de polluants d'un véhicule, comprenant les étapes suivantes :
- on exécute des calculs de cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
- on exécute simultanément des calculs des émissions de polluants, lesdits calculs permettant de déterminer les émissions de polluants à partir des quantités des composés chimiques calculées,

Pour ce procédé, plusieurs calculs de cinétique chimique sont exécutés simultanément.

De préférence le procédé est mis en oeuvre par le système tel que décrit ci-dessus.

### Présentation succincte des figures

D'autres caractéristiques et avantages du procédé selon l'invention, apparaîtront à la lecture de la description ci-après d'exemples non limitatifs de réalisations, en se référant aux figures annexées et décrites ci-après.
La figure 1 illustre l'architecture globale du système informatique selon l'invention.
La figure 2 illustre l'architecture du premier groupe de processeurs dédié à l'exécution de calculs de cinétique chimique selon l'invention.

### Description détaillée de l'invention

L'invention concerne un système informatique de prédiction des émissions de polluants d'un véhicule, comprenant plusieurs processeurs alloués pour la détermination des émissions de polluants. Le système informatique comprend en outre :
- un premier groupe de processeurs dédié à l'exécution de calculs de la cinétique chimique, lesdits calculs de la cinétique chimique permettant de déterminer des quantités de tous les composés chimiques présents dans une réaction chimique dans un cylindre d'un moteur dudit véhicule en fonction d'un schéma réactionnel donné et des conditions thermodynamiques dudit moteur ;
- un deuxième groupe de processeurs dédié à l'exécution de calculs des émissions de polluants, lesdits calculs permettant de déterminer les émissions de polluants à partir des quantités des composés chimiques,
les exécutions des calculs par lesdits deux groupes de processeurs étant simultanées.

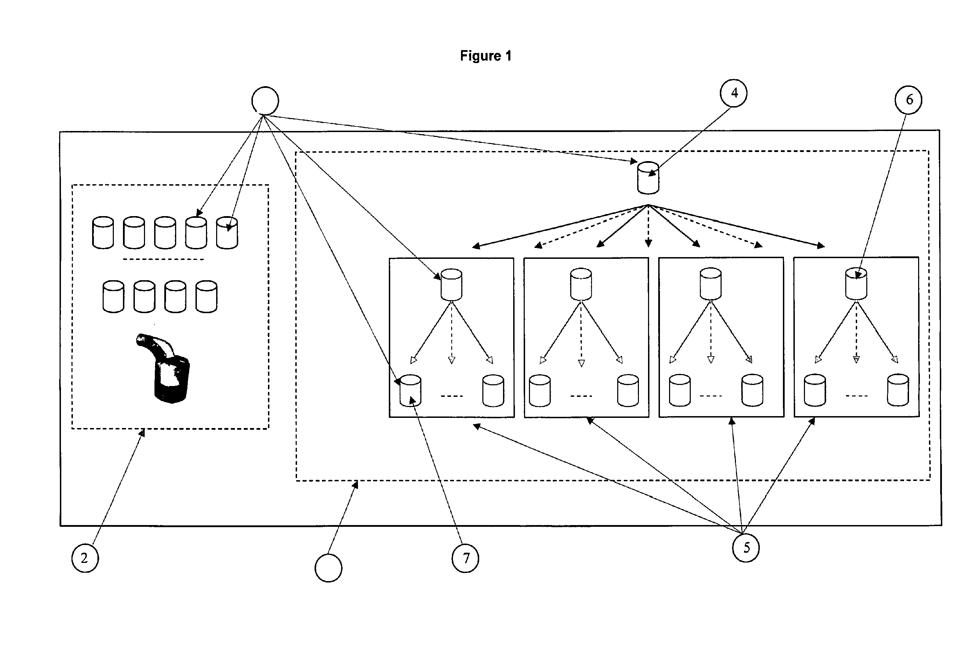

La figure 1 illustre la structure globale du système informatique selon l'invention. Le système possède une structure parallèle, permise par l'architecture parallèle des nouveaux calculateurs. Ainsi, les calculs de cinétique chimique sont exécutés lors des calculs des émissions de polluants. Ainsi le temps de simulation de la post-oxydation est réduit.

Afin de mettre en place le couplage entre les deux codes de calcul, le système selon l'invention comporte une architecture parallèle permettant de distribuer et de répartir automatiquement les calculs de cinétiques chimiques sur plusieurs processeurs (3) à la fois.

Dans un premier temps, cette architecture va diviser en deux groupes (1 ; 2) les processeurs alloués pour la simulation. Le premier groupe (1) est dédié à l'exécution des calculs de cinétique chimique. Lesdits calculs de la cinétique chimique permettent de déterminer des quantités molaires de tous les composés chimiques présents dans une réaction chimique dans le moteur du véhicule en fonction d'un schéma réactionnel donné et des conditions thermodynamiques du moteur. Le deuxième groupe (2) est réservé à l'exécution du code de calcul des émissions de polluants. Ces calculs permettent de déterminer les émissions de polluants à partir des quantités molaires simulées des composés chimiques. De cette façon, chaque partie de calcul est exécutée par des processeurs qui lui sont exclusivement réservés.

Dans un second temps, le premier groupe de processeurs (1) est divisé en plusieurs grappes de processeurs (5). En effet, le premier groupe de processeurs (1) comporte un processeur nommé superviseur global (4) et plusieurs grappes de processeurs (5). Le rôle du superviseur global (4) est de recevoir les informations parallèles du deuxième groupe de processeurs (2) et de distribuer les calculs sur les différentes grappes (5) afin d'optimiser le temps de calcul.

Selon un mode de réalisation préférentiel, au sein d'une grappe de processeurs (5), on dispose un superviseur local (6) qui reçoit des informations du superviseur global (4) qui va lui-même répartir les calculs sur des processeurs esclaves (7) inclus dans ladite grappe de processeurs (5).

Ce type d'architecture va permettre de distribuer les calculs de cinétique chimique sur un grand nombre de processeurs regroupés en grappes (5) afin de réduire les temps de calcul. D'autre part, les deux niveaux de superviseurs (4 ; 6) vont permettre de limiter des effets dits d'entonnoir liés au renvoi des données calculées en fin de calcul et d'automatiser la répartition de la charge des calculs sur les différents processeurs esclaves (7).

Les superviseurs (4 ; 6) distribuent les calculs respectivement sur les grappes de processeurs (5) et sur les processeurs esclaves (7) qui sont disponibles, c'est-à-dire ceux pouvant effectuer des calculs. Il s'agit d'une part des grappes dont au moins un processeur esclave est disponible, et d'autre part des processeurs esclaves non affectés à l'exécution d'un calcul. Ainsi, les calculs sont répartis de manière homogène sur les processeurs (3).

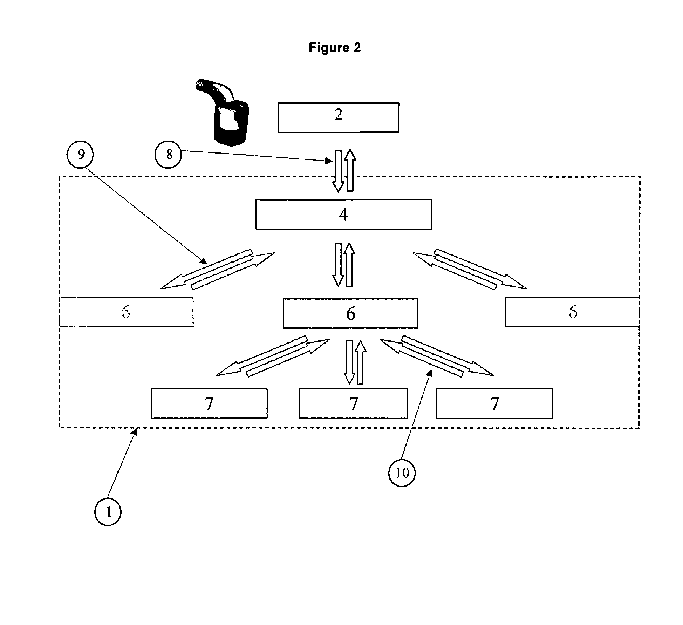

La figure 2 illustre la structure du premier groupe de processeurs, avec notamment une représentation des échanges de données entre les différents composants du système selon l'invention.

A chaque itération en temps, des données issues du calcul des émissions de polluants sont envoyées au superviseur global (4) par des premiers moyens de communication (8) disposés entre les deux groupes de processeurs (1 ; 2). Le superviseur global (4) réceptionne les paramètres pour réaliser le calcul de cinétique chimique et répartit automatiquement ces données sur les différentes grappes de processeurs (5) disponibles (c'est à dire sur lesquelles aucun calcul n'est en train d'être exécuté) par des deuxièmes moyens de communication (9). Les superviseurs locaux (6) des grappes de processeurs (5) distribuent les calculs sur les processeurs esclaves (7) disponibles par des troisièmes moyens de communication (10). Chaque superviseur local (6) envoie à chaque processeur esclave (7) un jeu de données contenant les paramètres d'une zone d'un modèle du moteur simulé. Dès qu'un processeur esclave (7) termine son calcul, celui-ci envoie le résultat du calcul de chimie au superviseur local (6) par les troisièmes moyens de communication (10). Le superviseur local (6) stocke le résultat, puis renvoie à ce processeur esclave (7) les paramètres concernant une autre zone du modèle du moteur à simuler. L'algorithme est terminé lorsqu'un calcul de chimie a été réalisé sur l'ensemble du modèle du moteur, c'est-à-dire l'ensemble des zones du modèle en provenance du code de calcul des émissions de polluants.

De préférence, le code de calcul dédié aux calculs des émissions de polluants utilise un modèle maillé de la chambre de combustion du moteur. Ainsi, une zone du modèle du moteur simulée par un processeur esclave (7) correspond à une maille ou à un paquet de mailles de ce modèle.

Cette méthode de distribution des calculs favorise une bonne répartition des charges sur tous les processeurs. Cela permet par conséquent de réduire le temps de simulation de la post-oxydation.

Dans un mode de réalisation préférentiel, le couplage direct des calculs des émissions de polluants et des calculs de cinétique chimique est implémenté en langage de programmation Fortran, de préférence en Fortran 90. Le couplage direct peut avantageusement utiliser la librairie de l'interface MPI (Message Passing Interface : interface de passage de message) pour permettre la distribution des calculs en parallèle et ainsi réduire le temps d'exécution du code. Les deux codes de calculs sont distincts et une allocation dynamique des processeurs est réalisée.

L'interface de passage des messages MPI est une norme définissant une bibliothèque de fonctions, utilisable avec les langages C et Fortran. Elle permet d'exploiter des ordinateurs distants ou multiprocesseurs par passage de messages. Cette technique est couramment utilisée pour l'exécution de programmes parallèles sur des systèmes à mémoire distribuée.

Cette interface MPI présente l'avantage d'obtenir de bonnes performances aussi bien sur des machines massivement parallèles à mémoire partagée que sur des ensembles d'ordinateurs hétérogènes à mémoire distribuée. En outre, elle est disponible sur de très nombreux matériels et systèmes d'exploitation. Ainsi, MPI possède l'avantage par rapport aux autres bibliothèques de passage de messages d'être grandement portable, car l'interface MPI a été implantée sur presque toutes les architectures de mémoires et est rapide car chaque implantation a été optimisée pour le matériel sur lequel il s'exécute.

Lorsqu'on utilise cette interface, on appelle communicateur un ensemble de processus pouvant communiquer ensemble, et deux processus ne pourront communiquer que s'ils sont dans un même communicateur. Un communicateur initial englobe tous les processus (MPI_COMM_WORLD), qu'il est possible de subdiviser en communicateurs plus petits correspondants à des entités logiques. Il existe deux types de communicateurs : les intracommunicateurs et les intercommunicateurs. Les intracommunicateurs sont les communicateurs standards, alors que les intercommunicateurs servent à créer un pont entre deux intracommunicateurs.

Ainsi, selon l'invention, la division en deux groupes de processeurs (1 ; 2) s'effectue grâce à la fonction MPI_COMM_SPLIT incluse dans la libraire de fonctions de l'interface MPI. Les processeurs dédiés aux calculs des émissions de polluants (2) sont inclus dans l'espace de communicateur global (2) de calcul des émissions de polluants et ceux en charge de l'exécution de la chimie (1) sont inclus dans l'espace de communicateur global (1) de calcul de la cinétique chimique. Des communications MPI sont créées entre ces deux espaces, ils correspondent aux premiers moyens de communication (8) décrits ci-dessus.

Selon l'invention, le communicateur de calcul de la cinétique chimique (1) est subdivisé en un superviseur global (4) et en au moins un communicateur de chimie (5), chaque communicateur de chimie (5) correspondant respectivement à une grappe de processeurs (5). Au sein de chaque communicateur de chimie (5), on retrouve un superviseur local (6) qui reçoit les informations du superviseur global (4) qui va lui-même répartir les calculs sur des processeurs esclaves inclus dans son communicateur.

Deux types de communications entre les différents communicateurs et au sein d'un même communicateur sont nécessaires pour diffuser les données. Il s'agit d'une part des communications inter-communicateur qui permettent de communiquer d'un communicateur à un autre. Par exemple, une communication inter-communicateur (9), correspondant aux deuxièmes moyens de communication (9), est créée entre le superviseur global (4) et chaque superviseur local (6) du communicateur de chimie (5) grâce à la fonction MPI_INTER_COMM_CREATE de la libraire de fonctions de l'interface MPI. D'autre part, des communications intra-communicateur (10), correspondant aux troisièmes moyens de communication (10), échangent des données entre un superviseur local (6) et un processeur esclave (7) au sein même d'un communicateur (5). Les transferts de données sont faits grâce à des communications MPI point-à-point ou collectives (par exemple fonction MPI_BCAST de la librairie de fonctions de l'interface MPI).

Les communications point-à-point permettent à deux processus à l'intérieur d'un même communicateur d'échanger une donnée sous forme scalaire, d'un tableau ou du type dérivé. Les fonctions de la libraire de l'interface MPI correspondantes sont MPI_SEND, MPI_RECV et MPI_SENDRECV.

Les communications collectives impliquent tous les processus d'un communicateur. Il est possible d'envoyer une même donnée à tous les processus (MPI_BCAST), de découper un tableau entre tous les processus (MPI_SCATTER), ou d'effectuer une opération (par exemple addition) à laquelle chaque processus contribue.

De préférence, le code de calcul utilisé pour les calculs des émissions de polluants est un programme de type CFD (pour Computational Fluid Dynamics, pouvant être traduit par mécanique des fluides numérique), on peut utiliser notamment le logiciel IFP-C3D ®, développé par IFP Energies Nouvelles. Grâce à ce logiciel, la simulation tridimensionnelle (3D) est utilisée pour calculer les écoulements diphasiques (gaz / carburant liquide) réactifs dans les moteurs à combustion interne. Ce logiciel permet d'analyser les phénomènes physiques complexes se produisant dans les moteurs (balayage lié au croisement de soupapes, formation de film liquide sur les parois, formation de polluants ...). Pour les calculs de cinétique chimique, on peut utiliser le logiciel Chemkin ®, développé par Reaction Design ®. Ce logiciel est apte à résoudre un nombre important de combinaisons de réactions chimiques dans le but de comprendre un problème complexe et est donc adapté à notre problématique.

L'invention concerne également un procédé de prédiction des émissions de polluants, dans lequel on réalise les étapes suivantes :
- on exécute des calculs de cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
- on exécute simultanément des calculs des émissions de polluants, lesdits calculs permettant de déterminer les émissions de polluants à partir des quantités des composés chimiques calculées.

Pour le procédé selon l'invention, plusieurs calculs de cinétique chimique sont exécutés simultanément.

Avantageusement le procédé est mis en oeuvre par le système tel que décrit ci-dessus.

## Revendications

1. Système de prédiction des émissions de polluants d'un véhicule équipé d'un moteur, comprenant plusieurs processeurs (3) alloués pour la détermination des émissions de polluants comprenant :
un premier groupe de processeurs (1) dédié à l'exécution de calculs de la cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
• un deuxième groupe de processeurs (2) dédié à l'exécution de calculs des émissions de polluants, lesdits calculs permettant de déterminer les émissions de polluants à partir des quantités des composés chimiques calculées,
les exécutions des calculs par lesdits deux groupes de processeurs (1 ; 2) étant simultanées,
**caractérisé en ce que** ledit premier groupe de processeurs (1) est constitué d'un processeur superviseur global (4) et d'au moins une grappe de processeurs (5), ledit superviseur (4) permettant le stockage de données nécessaires pour les calculs et de répartir lesdits calculs sur lesdites grappes (5) de processeurs disponibles.

2. Système selon la revendication 1, dans lequel ladite au moins une grappe de processeurs (5) est constituée d'un processeur superviseur local (6) et d'au moins un processeur esclave (7), ledit superviseur local (6) permettant de stocker les données et de répartir lesdits calculs entre lesdits processeurs esclaves (7), et chaque processeur esclave (7) étant apte à réaliser des calculs de cinétique chimique pour une portion du modèle dudit moteur.

3. Système selon l'une des revendications précédentes, dans lequel ledit système comprend des premiers moyens de communication (8) entre les deux groupes de processeurs (1 ; 2), lesdits moyens de communication (8) permettant de transférer les données issues du calcul des émissions de polluants vers le premier groupe de processeurs (1) ainsi que les résultats des calculs de cinétique chimique vers ledit deuxième groupe de processeurs (2).

4. Système selon l'une des revendications précédentes, dans lequel ledit système comprend des deuxièmes moyens de communication (9) entre ledit superviseur global (4) et lesdites grappes de processeurs (5), lesdits moyens de communication (9) permettant de transférer les données nécessaires pour les calculs depuis ledit superviseur global (4) vers lesdites grappes (5) ainsi que les résultats desdites simulations depuis lesdites grappes (5) vers ledit superviseur global (4).

5. Système selon la revendication 2, dans lequel ledit système comprend des troisièmes moyens de communication (10) entre lesdits superviseurs locaux et lesdits processeurs esclaves, lesdits moyens de communication (10) permettant de transférer les données nécessaires pour la simulation depuis ledit superviseur local (6) vers lesdits processeurs esclaves (7) ainsi que les résultats desdits calculs desdits processeurs esclaves (7) vers ledit superviseur local (6).

6. Système selon l'une des revendications précédentes, dans lequel lesdits calculs de la cinétique chimique sont exécutés par un logiciel de cinétique chimique, apte à être exécuté par le premier groupe de processeurs (1).

7. Système selon l'une des revendications précédentes, dans lequel lesdits calculs des émissions de polluants sont exécutés par un logiciel de calculs, apte à être exécuté par le deuxième groupe de processeurs (2).

8. Système selon l'une des revendications précédentes, dans lequel le couplage des calculs des deux groupes de processeurs (1 ; 2) utilise une interface de passage de messages MPI.

9. Système selon les revendications 3 à 5 et 8, dans lequel lesdits moyens de communications (8 ; 9 ; 10) sont réalisés par des fonctions choisies dans la librairie de fonctions de l'interface MPI.

10. Système selon l'une des revendications 8 et 9, dans lequel chaque groupe de processeurs (1 ; 2) constitue un communicateur global.

11. Système selon l'une des revendications 8 à 10, dans lequel chaque grappe de processeurs (5) constitue un communicateur de chimie.

12. Procédé de prédiction des émissions de polluants d'un véhicule, comprenant les étapes suivantes :
• on exécute des calculs de cinétique chimique, lesdits calculs de la cinétique chimique permettant de déterminer des quantités des composés chimiques présents dans une réaction chimique dans ledit moteur en fonction d'un schéma réactionnel donné et des conditions thermodynamiques dudit moteur ;
• on exécute simultanément des calculs des émissions de polluants, lesdits calculs permettant de déterminer les émissions de polluants à partir des quantités des composés chimiques calculées,
**caractérisé en ce que** plusieurs calculs de cinétique chimique sont exécutés simultanément.

13. Procédé selon la revendication 12, **caractérisé en ce qu'il** est mis en oeuvre par le système selon l'une des revendications 1 à 11.
